# EUROPEAN PATENT APPLICATION

(11) **EP 1 743 904 A2**
(43) Date of publication of application: **17.01.2007**
(21) Application number: 06116503.1
(22) Date of filing: 03.07.2006
(51) Int. Cl.: C07K 14/435

(54) **Guinea pig chymase**

(30) Priority: 13.07.2005 EP 05106404
(71) Applicant: F. HOFFMANN-LA ROCHE AG, 4070 Basel (CH)
(72) Inventor: Fingerle, Juergen, 79400 Kandern (DE); Beauchamp, Jeremy, 4125 Riehen (CH)

(57) **Abstract**

The present invention relates to the sequence of guinea pig chymase. Since guinea pigs express only one chymase isoform, they are particularly suitable as a model for a method for identifying compounds that modulate chymase.

SEQUENCE LISTING

<110>  F. Hoffmann-La Roche AG

<120>  guinea pig chymase

<130>  case 23232

<160>  21

<170>  PatentIn version 3.3

<210>  1
<211>  954
<212>  DNA
<213>  Cavia porcellus

<220>
<221>  guinea pig Chymase
<222>  (1)..(954)

<400>  1

agcacctgct gtgggcagcc tccctgagaa gatgtgtctt ctttctctcc ctctactact 60

ctttctccag tacaccagag ccaaagctgg ggaagtcatc ggtggcacag agtgcaagcc 120

acactcccgc ccctacatgg cttacctaga aattgtctct tctgagggat atgagaaaga 180

ctgtagtggt ttcttgatac gacggaattt tgtgctaaca gcagctcact gtgcaggaag 240

gtctctaaca gtcaacctag gggtccacaa caaaaaaatg aaagaagaca catggcagag 300

gcttaaggtt ataaagcaat tcctgcatcc aaactacaat agctctgtac ttctccacga 360

catcatgtta ctcaagttgg agaagaaagc caacctgacc ctagccgtgg gcacactccc 420

cctcccacct gaatgcaact tcctcacatc tggaagaatg tgccgagcag caggctgggg 480

gcgcacgaat gtggaggagc ctgcctccga cactcttcag gaggtgaagc tgagactcat 540

ggatccccaa gcctgcaaac acttcccaaa ctttaaccac aatctccagc tgtgtgtggg 600

taatcctagg aagagaaaat ctgtattcaa gggagactct ggaggtcccc tcctgtgtgc 660

agggatagcc cagggcattg tatcctatgc acatcggaat gcaaaacctc ctgttgtctt 720

cacccgaatc tcccattacc ggccgtggat caacaagatc ttgaaagcaa attaaccatg 780

gagcctcggc cagccctgaa aggaaatctg gaactggatc tgagcaggtt ttctgtacca 840

cttactctgg ccctgtctct ggttcctgtt gaagccagcc catgtccttg agactccaga 900

aagtccctac aactcataga actctcaata aagcttcagc aaaaatccca aaaa 954

<210> 2

<211> 247

<212> PRT

<213> Cavia porcellus

<220>

<221>  guinea pig Chymase

<222>  (1)..(247)

<400>  2

Met Cys Leu Leu Ser Leu Pro Leu Leu Leu Phe Leu Gln Tyr Thr Arg

1               5                   10                  15

Ala Lys Ala Gly Glu Val Ile Gly Gly Thr Glu Cys Lys Pro His Ser

            20                  25                  30

Arg Pro Tyr Met Ala Tyr Leu Glu Ile Val Ser Ser Glu Gly Tyr Glu

        35                  40                  45

Lys Asp Cys Ser Gly Phe Leu Ile Arg Arg Asn Phe Val Leu Thr Ala

    50                  55                  60

Ala His Cys Ala Gly Arg Ser Leu Thr Val Asn Leu Gly Val His Asn

65                  70                  75                  80

Lys Lys Met Lys Glu Asp Thr Trp Gln Arg Leu Lys Val Ile Lys Gln

                85                  90                  95

Phe Leu His Pro Asn Tyr Asn Ser Ser Val Leu Leu His Asp Ile Met

            100                 105                 110

Leu Leu Lys Leu Glu Lys Lys Ala Asn Leu Thr Leu Ala Val Gly Thr

        115                 120                 125

Leu Pro Leu Pro Pro Glu Cys Asn Phe Leu Thr Ser Gly Arg Met Cys

    130                 135                 140

Arg Ala Ala Gly Trp Gly Arg Thr Asn Val Glu Glu Pro Ala Ser Asp
145                 150                 155                 160
Thr Leu Gln Glu Val Lys Leu Arg Leu Met Asp Pro Gln Ala Cys Lys
                165                 170                 175
His Phe Pro Asn Phe Asn His Asn Leu Gln Leu Cys Val Gly Asn Pro
            180                 185                 190
Arg Lys Arg Lys Ser Val Phe Lys Gly Asp Ser Gly Gly Pro Leu Leu
        195                 200                 205
Cys Ala Gly Ile Ala Gln Gly Ile Val Ser Tyr Ala His Arg Asn Ala
    210                 215                 220
Lys Pro Pro Val Val Phe Thr Arg Ile Ser His Tyr Arg Pro Trp Ile
225                 230                 235                 240
Asn Lys Ile Leu Lys Ala Asn
                245

<210>  3
<211>  33
<212>  DNA
<213>  Artificial

<220>
<223>  derived from guinea pig

<220>

<221>  GP_CHY_12_F

<222>  (1)..(33)

<220>

<221>  misc_feature

<222>  (8)..(9)

<223>  n is a, c, g, or t

<220>

<221>  misc_feature

<222>  (12)..(12)

<223>  n is a, c, g, or t

<220>

<221>  misc_feature

<222>  (21)..(21)

<223>  n is a, c, g, or t

<400>  3

garkbyannc cncaytcccg nccytatcat ggc                                    33

<210> 4

<211> 31

<212> DNA

<213> Artificial

<220>

<223> derived from guinea pig sequence

<220>

<221> GP_CHY_13_F

<222> (1)..(31)

<220>

<221> misc_feature

<222> (8)..(9)

<223> n is a, c, g, or t

<220>

<221> misc_feature

<222> (12)..(12)

<223> n is a, c, g, or t

<220>

<221>  misc_feature

<222>  (21)..(21)

<223>  n is a, c, g, or t

<400>  4

garkbyannc cncaytcccg nccytatcat g                                    31

<210>  5

<211>  29

<212>  DNA

<213>  Artificial

<220>

<223>  derived from guinea pig sequence

<220>

<221>  GP_CHY_14_F

<222>  (1)..(29)

<220>

<221>  misc_feature

<222>  (8)..(9)

<223>  n is a, c, g, or t

<220>

<221>  misc_feature

<222>  (12)..(12)

<223>  n is a, c, g, or t

<220>

<221>  misc_feature

<222>  (21)..(21)

<223>  n is a, c, g, or t

<400>  5

garkbyannc cncaytcccg nccytatca                                          29

<210>  6

<211>  31

<212>  DNA

<213>  Artificial

<220>

<223>  derived from guinea pig sequence

<220>

<221>  GP_CHY_15_F

<222>  (1)..(31)

<400>  6

gagtcaaagc cacactcccg cccttacatg g                                    31

<210>  7

<211>  31

<212>  DNA

<213>  Artificial

<220>

<223>  derived from guinea pig sequence

<220>

<221>  GP_CHY_16_F

<222>  (1)..(31)

<400>  7

gagtgcagac cacatgcccg cccctacatg g                                    31

<210>  8

<211>  29

<212>  DNA

<213>  Artificial

<220>

<223>  derived from guinea pig sequence

<220>

<221>  GP_CHY_17_R

<222>  (1)..(29)

<220>

<221>  misc_feature

<222>  (15)..(15)

<223>  n is a, c, g, or t

<220>

<221>  misc_feature

<222>  (18)..(18)

<223>  n is a, c, g, or t

<400>  8

kyrcacasda rdggnccncc dgagtcycc                                         29

<210>  9

<211>  29

<212>  DNA

<213>  Artificial

<220>

<223>  derived from guinea pig sequence

<220>

<221>  GP_CHY_18_R

<222>  (1)..(29)

<220>

<221>  misc_feature

<222>  (15)..(15)

<223>  n is a, c, g, or t

<220>

<221>  misc_feature

<222>  (18)..(18)

<223>  n is a, c, g, or t

<400>  9

kyrcacasda rdggnccncc dgagtcycc                                          29

<210>  10

<211>  24

<212>  DNA

<213>  Artificial

<220>

<223>  derived from guinea pig sequence

<220>

<221>  misc_feature

<222>  (15)..(15)

<223>  n is a, c, g, or t

<220>

<221>  misc_feature

<222>  (18)..(18)

<223>  n is a, c, g, or t

<400>  10

kyrcacasda rdggnccncc dgag                                              24

<210>  11

<211>  29

<212>  DNA

<213>  Artificial

<220>

<223>  derived from guinea pig sequence

<220>

<221>  GP_CHY_20_R

<222>  (1)..(29)

<400>  11

gcacacagaa gaggtccccc ggagtctcc                                         29

<210>  12

<211>  29

<212>  DNA

<213>  Artificial

<220>

<223>  derived from guinea pig sequence

<220>

<221>  GP_CHY_21_R

<222>  (1)..(29)

<400>  12

ttacacacaa gaggccctcc agagtcccc                                         29

<210>  13

<211>  28

<212>  DNA

<213>  Artificial

<220>

<223>  derived from guinea pig sequence

<220>

<221>  CHYGP_22_F

<222>  (1)..(28)

<400>  13

gagaaagact gtagtggttt cttgatac                                          28

<210>  14

<211>  28

<212>  DNA

<213>  Artificial

<220>

<223>  derived from guinea pig sequence

<220>

<221>  CHYGP_23_R

<222>  (1)..(28)

<400>  14

gtatcaagaa accactacag tctttctc                                          28

<210>  15

<211>  24

<212>  DNA

<213>  Artificial

<220>

<223>  derived from guinea pig sequence

<220>

<221>  CHYGP_24_F

<222>  (1)..(24)

<400>  15

atgctgcttc ttcctctccc cctg                                              24

<210>  16

<211>  20

<212>  DNA

<213>  Artificial

<220>

<223>  derived from guinea pig sequence

<220>

<221>  GP_CHY_25_F

<222>  (1)..(20)

<400>  16

ctaacagtca acctaggggt                                                    20

<210>  17

<211>  20

<212>  DNA

<213>  Artificial

<220>

<223>  derived from guinea pig sequence

<220>

<221>  GP_CHY_26_F

<222>  (1)..(20)

<400>  17

agctcactgt gcaggaaggt                                                    20

<210>  18

<211>  20

<212>  DNA

<213>  Artificial

<220>

<223>  derived from guinea pig sequence

<220>

<221>  GP_CHY_27_F

<222>  (1)..(20)

<400>  18

gacggaattt tgtgctaaca                                                    20

<210>  19

<211>  20

<212>  DNA

<213>  Artificial

<220>

<223>  derived from guinea pig sequence

<220>

<221>  GP_CHY_28_R

<222>  (1)..(20)

<400>  19
   acccctaggt tgactgttag                                                    20

<210>  20

<211>  20

<212>  DNA

<213>  Artificial

<220>

<223>  derived from guinea pig sequence

<220>

<221>  GP_CHY_29_R

<222>  (1)..(20)

<400>  20

accttcctgc acagtgagct                                                    20

<210>  21

<211>  20

<212>  DNA

<213>  Artificial

<220>

<223>  derived from guinea pig sequence

<220>

<221>  GP_CHY_30_R

<222>  (1)..(20)

<400>  21

tgttagcaca aaattccgtc                                                    20

## Description

The present invention relates to guinea pig chymase, the nucleotide sequence that encodes the guinea pig chymase, and methods for identifying compounds that inhibit the activity of chymase and PAOD (peripheral arterial occlusive disease) and counteract asthma, based on the guinea pig chymase.

Chymase is a chymotrypsin-like serine protease which, with the exception of the rat, occurs in the secretory granules of mast cells. In immunology, mast cells are known above all for their role in allergic and fibrotic reactions. If the mast cells are activated, then chymase is released together with histamine and many other mediators via degranulation into the extracellular space. After its release, chymase develops a range of important inflammation-promoting effects. Chymase splits various physiologically and pathologically important proteins, such as e.g. the stem cell factor, big endothelin, TGF beta or apolipoproteins, and is also capable of activating collagenase proteolytically. In addition to these effects, it is assumed that one of the main tasks of human chymase is the production of angiotensin II from angiotensin I. For this reason, it is also speculated that chymase plays an important role in the case of heart failure, high blood pressure, allergy, dermatitis, rheumatic arthritis, asthma and chronic inflammations.

Species such as humans have only one chymase gene, whereas other species have several. It appears as though chymases are very similar to one another if only one is contained in an organism. If several are present, then there appears to be a division of tasks and adaptation to these. Thus animal models with just one chymase are the most suitable for clinical tests, as humans likewise only have one chymase. In the case of rodents in particular, such as mice and rats, we find numerous chymase genes, and even in hamsters we find two.

It is an object of the present invention to find a species of animal that has only a single chymase isoform which at the same time is highly homologous to human chymase.

The present invention achieves this through the cloning and provision of guinea pig chymase. For this, both genomic and total RNA were isolated from guinea pig organ samples, in particular from the heart. In order to be able to duplicate chymase or chymase-like sequences in a targeted manner, it was necessary to develop primers with a specificity in respect of these sequences. The primers that were created here are listed in table 5. With these primers, PCR amplifications starting out from genomic DNA were carried out. In order to enable a statement to be made about the results of the PCRs, the sequences of the plasmid inserts had to be determined. Via a database search, these were then compared with all DNA sequences known to date, and examined for homologies. It was also possible thus to convert the DNA sequences into an amino acid sequence of the resulting protein.

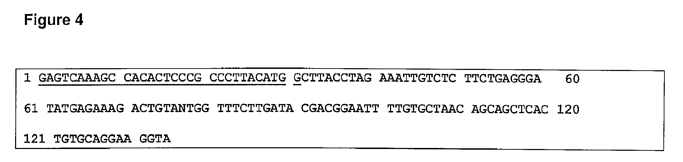

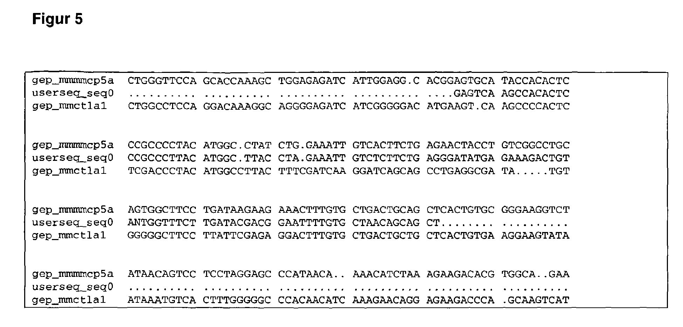

The sequencing of 11 samples yielded the DNA sequences of serine proteases that are already known and are very similar to chymase. However for one sample, for the first time a chymase-like guinea pig part-sequence was found which is shown in figure 4. The underlined bases here correspond to the part encoded by the primer. A search in relation to homologies to other DNA sequences showed that this sequence was very similar to DNA molecules already known, such as e.g. the granzyme (gep_mmmmcp5a) or the chymase 1 (gep_mmctala1) of the mouse, but there was no complete agreement. Such a sequence comparison is shown in figure 5.

The batches that yielded results contained the primers GP_CHY_12_F and GP_CHY_17_R, and the primers GP_CHY_15_F and GP_CHY_17_R. It would thus appear that of all the primers developed so far, the primer GP_CHY_17_R has the highest specificity in relation to chymase-like sequences in guinea pigs.

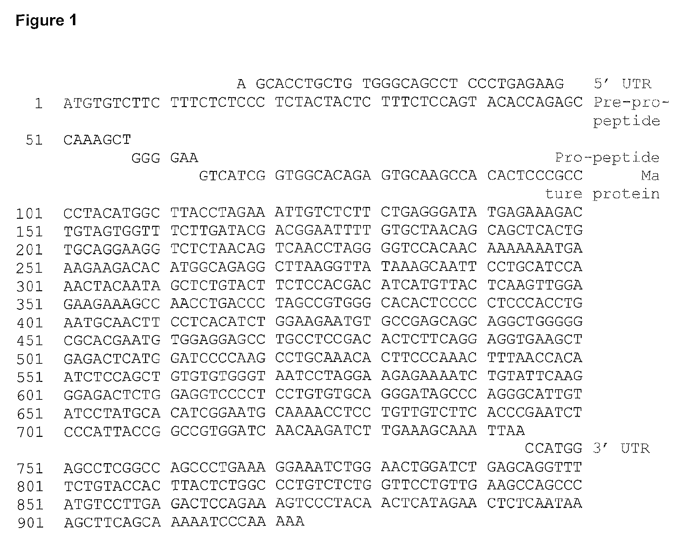

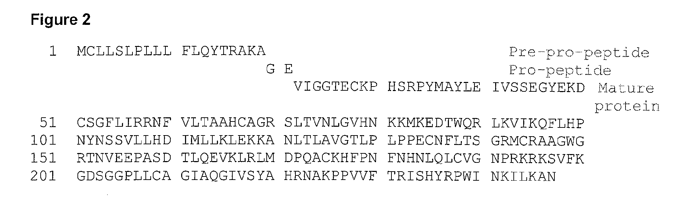

In a next step, on the basis of the sequence isolated from the genomic DNA, the encoded sequence was determined, in that a further fragment of the guinea pig chymase was amplified using specific primers (table 6) from guinea pig cDNA as a template. Starting out from this part-sequence, further primers were selected (table 7), in order to determine a further cDNA sequence of guinea pig chymase. In various reactions, it was possible to determine a further sequence, which is shown in figure 1 (Seq ID No. 1). Translation of the nucleic acid sequence in figure 1 yields the polypeptide sequence in figure 2 (Seq ID No. 2) of the mature chymase, without the signal peptide.

Altogether, 437 sequencings were carried out. In the course of this, guinea pig chymase was found 7 times, granzyme 7 times, but no alternative chymases were found.

The invention accordingly relates to a nucleic acid sequence comprising the sequence of Seq ID No. 1. For preference, the nucleic acid sequence is the sequence of Seq ID No. 1. The present invention furthermore relates to a polypeptide sequence comprising the sequence of Seq ID No. 2, wherein the polypeptide sequence is preferably the sequence of Seq ID No. 2. The present invention furthermore relates to a nucleic acid sequence comprising a nucleic acid sequence that encodes the polypeptide sequence named above. Furthermore, the present invention relates to an expression vector that comprises the aforementioned nucleic acid sequences. Moreover, the present invention relates to a host cell that comprises the aforementioned expression vector.

The guinea pig chymase polypeptide sequence was compared with known sequences (tables 1 and 2).

**Table 1: Comparison of known chymase sequences with the mature guinea pig chymase polypeptide sequence**

| Chymase | Abbreviation | Identity (%) | Homology (%) |
|---|---|---|---|
| Human | sw:mcpt1_human | 77.9 | 86.3 |
| Rat 1 | sw:mcpt1_rat | 57.1 | 72.1 |
| Hamster 1 | tr:o08732_mesau | 59.5 | 74.9 |
| Hamster 2 | tr:o70164_mesau | 72.6 | 85.8 |
| Mouse 1 | sw:mcpt5_mouse | 77.0 | 86.3 |

**Table 2: Comparison with the mature human chymase polypeptide sequence**

| Chymase | Abbreviation | Identity (%) | Homology (%) |
|---|---|---|---|
| Guinea pig | xxxx | 77.9 | 86.3 |
| Rat 1 | sw:mcpt1_rat | 57.5 | 72.9 |
| Hamster 1 | tr:o08732_mesau | 59.9 | 74.3 |
| Hamster 2 | tr:o70164_mesau | 70.8 | 81.9 |
| Mouse 1 | sw:mcpt5_mouse | 75.2 | 82.7 |

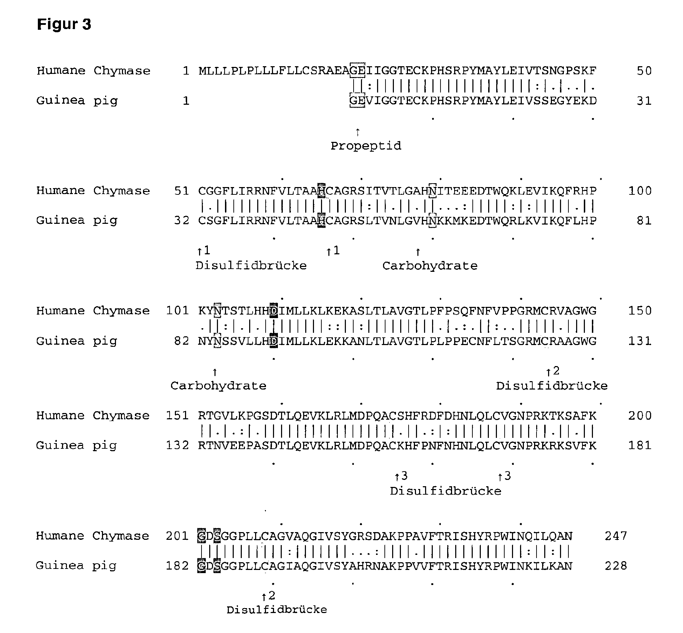

Of the animal species that are compared here, the guinea pig is the one with the highest identity and homology to human chymase (77.9 and 86.3 %). The chymases of human, mouse and guinea pig are so similar to one another that all these chymases are α chymases. Figure 3 shows that the guinea pig chymase contains all the important features of chymase:
- The disulphide bridges are at identical positions
- The positions of the active triangle are likewise identical
- Both sequences have a glycine at the entrance to pocket 1
- The carbohydrates agree

Since the guinea pig is the most suitable animal model, the present invention relates to a guinea pig with somatic cells and germ cells with a mutated chymase gene, wherein at least one allele is functionally interrupted through homologous recombination of the mutated chymase gene, wherein this functional interruption inhibits the expression of the functional chymase gene in the guinea pig cells.

The present invention thus also relates to the following methods for identifying modulators of chymase activity and inhibitors of PAOD:

A method is disclosed for screening compounds that modulate the activity of chymase, comprising the steps: a) bringing a compound into contact with isolated guinea pig chymase polypeptide according to claim 3 or 4; and b) determining the activity of guinea pig chymase; wherein a compound that inhibits or stimulates the activity of the guinea pig chymase is a compound that modulates the activity of the guinea pig chymase.

Also disclosed is a method for identifying compounds that modulate the activity of chymase, comprising the steps: a) bringing a compound into contact with isolated polypeptide according to claim 3 or 4; and b) determining the activity of guinea pig chymase; wherein a compound that inhibits or stimulates the activity of the guinea pig chymase is a compound that modulates the activity of the guinea pig chymase.

A further method is a method for identifying compounds that modulate the activity of chymase, comprising the steps: a) bringing a compound into contact with the host cell according to claim 7; and b) determining the activity of the guinea pig chymase that is expressed in the host cell; wherein a compound that inhibits or stimulates the activity of the guinea pig chymase is a compound that modulates the activity of the guinea pig chymase.

The present invention also discloses a method for identifying compounds that modulate the activity of chymase, comprising the steps: a) administering a compound to guinea pigs; and b) determining the activity of the guinea pig chymase; wherein a compound that inhibits or stimulates the activity of the guinea pig chymase is a compound that modulates the activity of the guinea pig chymase.

Finally, the present invention relates to a method for identifying compounds that inhibit PAOD, comprising the steps: a) administering to guinea pigs a compound that inhibits the guinea pig chymase; b) causing PAOD, and c) determining the extent of PAOD; wherein a compound that inhibits PAOD is a compound that leads to a reduction in the extent of the PAOD that is caused by step b). Preferably, PAOD is caused by administering lauric acid, through damage to a vessel with a balloon catheter or by feeding a diet with a high proportion of fat. Causing PAOD by administering lauric acid is particularly preferred. Another means of causing PAOD that is especially preferred is through damage to a vessel of the guinea pig with a balloon catheter, wherein the vessel is preferably the carotid.

The extent of PAOD is determined using familiar methods. For example, the extent of PAOD is determined by determining the formation of a neointima following a balloon catheter injury e.g. in the carotid of guinea pigs. Another possibility is determining the fatigue of guinea pigs running on a treadmill in the lauric acid model (Kawamura *et al.,* 1985, *Arzneimittelforschung* 35/7A: 1154-1156).

The present invention relates to a method for identifying compounds to combat asthma, comprising the steps: a) administering to guinea pigs a compound that inhibits the guinea pig chymase; b) causing asthma; and c) determining the extent of asthma, wherein a compound that inhibits asthma is a compound that leads to a reduction in the extent of the asthma that is caused by step b). In this method, familiar methods are used for inducing asthma and for determining the extent of asthma.

The present invention also relates to sequences, methods and guinea pigs as described above, in particular in relation to the following examples.

### Brief description of the figures:

- Figure 1:: Nucleic acid sequence of the guinea pig chymase (Seq ID No. 1)
- Figure 2:: Amino acid sequence of the mature guinea pig chymase (Seq ID No. 2)
- Figure 3:: Comparison of human chymase and guinea pig chymase
- Figure 4:: First sequence of the guinea pig chymase isolated from genomic DNA
- Figure 5:: Sequence comparison of mouse chymases with the cloned guinea pig sequence
- Figure 6:: Position of the primers for amplification and cloning of the guinea pig chymase cDNA
- Figure 7:: Guinea pig sequence from cDNA

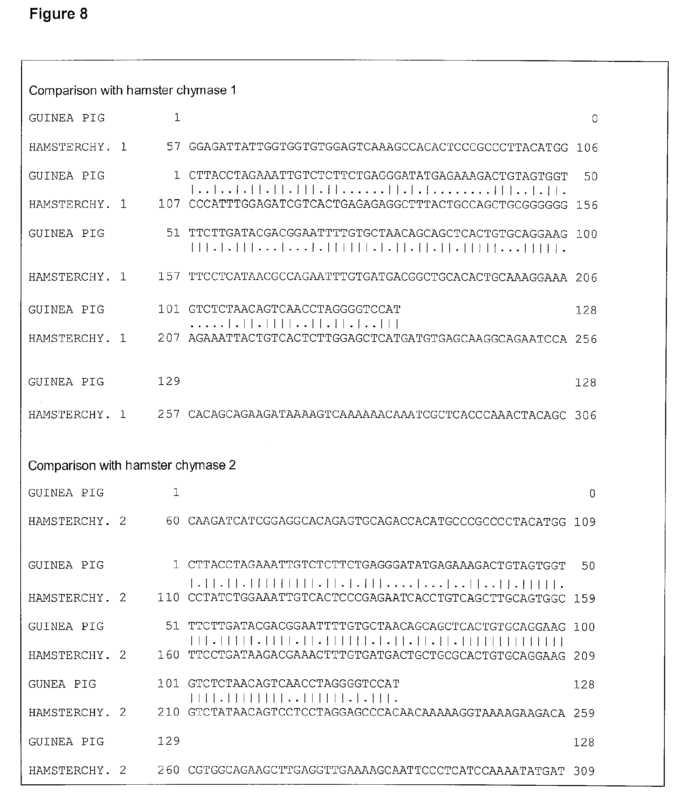

- Figure 8:: Comparison of the guinea pig chymase with hamster chymase 1 and 2
- Figure 9:: Position of the primers for cloning of a further sequence of the guinea pig chymase.

### Examples:

### Example 1: Cell disintegration and obtaining RNA from a guinea pig heart

In order to be able to demonstrate the existence and activity of chymase in guinea pigs, total RNA was isolated. The heart of a guinea pig was used as the organ sample.

### Example 1.1: Cell disintegration for obtaining RNA

Disintegration of the cells took place through the use of so-called Lysing Matrix D Column. For this, 5x10 mg and 5x30 mg of the heart of a guinea pig were filled respectively into tubes with small ceramic balls and overlaid with 300 µl of a mixture of RLT buffer (RNeasy kit) and β-mercaptoethanol (10 µl β-mercaptoethanol per 1 ml of RLT buffer). Subsequently, the tubes were shaken for 2x20 seconds with a FastPrep homogeniser, through which disintegration of the cells was effected through shearing forces. Through centrifugation, the coarsest cell debris was sedimented and the supernatant fluid was transferred into clean tubes.

Subsequently, 590 µl of distilled water and 10 µl 1 mg/ml protein kinase K were added to each tube, the solutions were mixed through inverting and incubated for 10 minutes at 55°C to break down the proteins. Through centrifugation for 3 minutes at 10,000 rpm and at room temperature, the coarsest cell debris was sedimented and 900 µl of the supernatant fluid was transferred into clean tubes.

### Example 1.2: RNA isolation

The isolation of the total RNA from this mixture of proteins and other cell components was carried out using the RNeasy Mini Kit in accordance with the instructions (see Manual RNeasy Kit, protocol for "Isolation of Total RNA from Heart, Muscle and Skin Tissue").
1. Add 450 µl of 100% EtOH each per batch for denaturing the RNA, mixing the solutions through inverting.
2. Bind the RNA to the silica membrane of the RNeasy columns with collecting vessel by filling the columns with, respectively, 700 µl of this mixture and centrifuging for 30 seconds at 10,000 rpm. and 4°C, discarding the through-flow.
3. Fill the columns with, respectively, 350 µl RW1 buffer and centrifuge for 30 seconds at 10,000 rpm and 4°C to wash the columns, discarding the through-flow.
4. Transfer the columns to new collecting vessels, filling the columns with, respectively, 500 µl RPE buffer and centrifuging for 30 seconds at 10,000 rpm and 4°C to wash the column.
5. Fill the columns with a further 500 µl RPE buffer and centrifuge for 2 minutes at 10,000 rpm and 4°C, in order to dry the silica membrane.
6. Transfer the columns to clean 1.5 ml tubes.
7. Add 30 µl RNase free water per column and subsequently elute the RNA through centrifugation for 30 seconds at 13,000 rpm and 4°C.

The concentrations of the various resulting RNA solutions were subsequently determined photometrically, at a wavelength of 260 nm.

### Example 1.3: Production of cDNA from guinea pig RNA

The production of cDNA was carried out by means of the Transcriptor First Strand cDNA Synthesis Kit according to the instructions, as described below. Here, two different batches were prepared, for the use of two different primers.
1. Pipette together:
   8 µl RNA 30.1
   1 µl oligo (dT) primer or 2 µl adapter primer (Invitrogen)
   Fill up with H₂O to 13 µl
2. Incubate the batches for 10 minutes at 65°C for adding the primers on the RNA.
3. Cool the batches on ice for 2 minutes.
4. Per batch, add:
   4 µl 5x reaction buffer
   0.5 µl *RNase* Inhibitor
   2 µl dNTPs
   0.5 µl *reverse transcriptase*
5. Incubate the batches for 1 hour at 50°C for the procedure of the *reverse transcriptase* reaction.
6. Incubate for 5 minutes at 85°C to inactivate the *reverse transcriptase.*
7. Cool the batches on ice for 2 minutes.

To remove low-molecular fragments and unused constituents of the RT-PCR, the different batches were purified by means of the QIAquick PCR Purification Kit according to the instructions for microcentrifuges, as follows:
1. Add 250 µl of PB buffer per RT-PCR batch, mixing the solutions.
2. Fill the QIAquick columns with collecting containers with one batch each and centrifuge for 1 minute at 13,000 rpm and 4°C to bind the DNA to the silica membrane of the column, discarding the through-flow.
3. To wash the columns, add in each case 750 µl PE buffer and centrifuge for 1 minute at 13,000 rpm and 4°C, discarding the through-flow.
4. Centrifuge the columns for 1 minute at 13,000 rpm and 4°C to dry the silica membrane.
5. Transfer the columns into clean 1.5 µl tubes.
6. Add 30 µl EB buffer per column, incubate for 1 minute at room temperature and subsequently elute the cDNA through centrifugation for 1 minute at 13,000 rpm and 4°C.

### Example 2: Cell disintegration and obtaining genome DNA from guinea pig heart

Another possibility for demonstrating chymase in guinea pigs was the use of genome DNA. This method was used since it could not be assumed with certainty that the gene that was sought was in fact expressed.

### Example 2.1: Cell disintegration

To obtain genome DNA, as in the case of RNA, disintegration of the cells was necessary. Once again, this was carried out via the aforementioned use of Lysing Matrix D columns. For this, 2x100 of the heart of a guinea pig were filled respectively into tubes with small ceramic balls and overlaid with 1.2 µl of digestion buffer. The composition of this buffer is shown in table 2-13.

**Table 3: Digestion buffer**

| **Component** | **Concentration** |
|---|---|
| NaCl | 100 mM |
| Tris-HCl pH 8.0 | 10 mM |
| EDTA pH 8.0 | 25 mM |
| SDS | 0.5% (w/v) |
| Protein kinase K | 0.1 mg/ml |

Subsequently, the tubes were shaken for 2x20 seconds with a FastPrep homogeniser, through which disintegration of the cells was effected through shearing forces.

### Example 2.2: Isolation of the DNA via phenol extraction and ethanol precipitation

The separation of proteins and DNA took place via phenol extraction by means of Phase Lock Gel according to the instructions, as follows.
1. Centrifuge the gel down in the vessel (2.0 ml) at 13,000 rpm for 20-30 seconds.
2. Add 100-750 µl disintegrated cells per vessel.
3. Add the same volume of organic solvent, in this case phenol: chloroform : isoamyl alcohol (25:24:1).
4. Mix for 10 minutes through occasional inversion (no vortexes!)
5. Centrifuge for phase separation at 13,000 rpm for 5 minutes.
6. Take off the supernatant fluid and process further.

Subsequently, the DNA was cleansed and concentrated via ethanol precipitation, as detailed below.
1. Transfer upper phase after phenol extraction into a new cup and add 1/10 of its own volume of 3 M sodium acetate (NaAc) pH 5.0 and 2.5 of its own volume of 100% EtOH.
2. Fish out the denatured DNA molecules with a sterile inoculating loop and transfer into a new cup.
3. Renature the DNA in 1.5 ml 10 mM tris-HCl pH 7.5 and repeat the addition of EtOH NaAc.
4. Centrifuge for 15 minutes at 11,200 rpm and 4°C.
5. Remove the supernatant fluid and resuspend the pellet in 1.5 ml 75% EtOH.
6. For salt removal, invert the suspension for 10 minutes.
7. Centrifuge for 10 minutes at 11,200 rpm and 4°C, remove supernatant fluid and air-dry the pellet for 10-15 minutes.
8. Dissolve the pellet in 1.5 ml 10 mM tris-HCl pH 7.5.

### Example 3: Search for chymase coding sequences in genome DNA

In order to be able to identify chymase sequences where the respective gene is inactive, it is necessary to multiply these sequences from genome DNA templates by means of a PCR. In this part-experiment, different degenerated primers which have a specificity for guinea pig chymase were developed and used for this purpose.

### Example 3.1: Development of primers for guinea pig chymase

It was assumed that chymase showed great homology in all animal models. Thus starting from the sequence for hamster chymase 1, which was already known, a database search was started for other known DNA sequences with as much in common as possible. The positive sequences of this search, which included e.g. many serine proteases and chymases of mice or rats, were then compared with one another, and from the regions with the widest variations between the individual sequences, primers were developed of which it could be assumed that they would bind chymase or chymase-like sequences. Within the individual primers, to some extent variations were allowed, in order to obtain a greater selection of sequences. These variations were produced by allowing several different bases at individual positions of the sequences. Such primers are termed degenerated. The variations were expressed via the generally applicable letter codes shown in table 4; production of these primers was undertaken by a contract firm.

**Table 4: Letter codes**

| **Letter** | **Possible bases** |
|---|---|
| B | C, G or T |
| D | A,G or T |
| H | A,C or T |
| K | G or T |
| M | A or C |
| N | A, C, G or T |
| R | A or G |
| S | C or G |
| V | A, C or G |
| W | A or T |
| Y | C or T |

**Table 5: Primers developed**

| **Primer name** | **Sequence** | **Transcription direction** |
|---|---|---|
| GP_CHY_12_F | GARKBYANNCCNCAYTCCCGNCCYTATCATGGC | Forward (seq ID 3) |
| GP_CHY_13_F | GARKBYANNCCNCAYTCCCGNCCYTATCATG | Forward (seq ID 4) |
| GP_CHY_14_F | GARKBYANNCCNCAYTCCCGNCCYTATCA | Forward (seq ID 5) |
| GP_CHY_15_F | GAGTCAAAGCCACACTCCCGCCCTTACATGG | Forward (seq ID 6) |
| GP_CHY_16_F | GAGTGCAGACCACATGCCCGCCCCTACATGG | Forward (seq ID 7) |
| GP_CHY_17_R | KYRCACASDARDGGNCCNCCDGAGTCYCC | Reverse (seq ID 8) |
| GP_CHY_18_R | KYRCACASDARDGGNCCNCCDGAGTC | Reverse (seq ID 9) |
| GP_CHY_19_R | KYRCACASDARDGGNCCNCCDGAG | Reverse (seq ID 10) |
| GP_CHY_20_R | GCACACAGAAGAGGTCCCCCGGAGTCTCC | Reverse (seq ID 11) |
| GP_CHY_21_R | TTACACACAAGAGGCCCTCCAGAGTCCCC | Reverse (seq ID 12) |

### Example 3.2: PCR with the primers that were produced

Through the use of the degenerated primers, the attempt was to be made to amplify chymase or chymase-like sequences. For this purpose, each of the F primers was combined with each R primer.

The PCR batches were composed as follows:
1 µl template (genome DNA)
1 µl 1^{st} primer (GP_CHY_12_F to 16_F)
1 µl 2^{nd} primer (GP_CHY_17_F to 21_R)
20 µl HotStarTaq Master Mix
17 µl H₂O

The PCR proceeded according to the following program:
- 95°C: 10 minutes

95°C 30 seconds
55°C 30 seconds 30 cycles
72°C 2 minutes 30 seconds

- 72°C: 10 minutes
- 4°C: storage

The PCR products were analysed with a 1% agarose / EtBr gel.

### Example 4: Multiplication of the PCR products that have been obtained

In order to be have sufficient material in respect of PCR products that have been obtained available for further investigations, these were amplified by means of transformation and via the cultivation of bacterial cultures.

### Example 4.1: Purification of the PCR batches

In order to remove low-molecular fragments and other interference substances for the later transformation, selected PCR batches were purified with the QIAquick PCR Purification Kit according to the instructions, as described above.

### Example 4.2: Transformation with the TOPO TA Cloning Kit

Since several products or low-molecular interference fragments frequently occur in PCR batches, in most cases direct sequencing is not possible. For this reason, for separation and amplification, the PCR products were incorporated into bacterial vectors and the plasmids were subsequently introduced into competent cells. This was carried out by means of the TOPO TA Cloning Kit, as described below.
1. Mix 4 µl PCR batch, 1µl saline solution and 1 µl TOPO vector. Incubate the batches for 5 minutes at room temperature.
2. Add 2.5 µl of this mixture to a tube of competent TOP 10 F' cells which were thawed on ice.
3. Incubate the cells for 5 minutes on ice, to take the plasmids into the cells.
4. Heat shock for 30 seconds at 42°C to stop DNA uptake by the cells, and abrupt cooling off on ice for 2 minutes.
5. Add 300 µl LB medium per bacteria tube and incubate for 1 hour at 37°C on the shaker.
6. Per batch, inoculate two LB/amp plates via plating out the bacteria, and incubate overnight at 37°C.

### Example 4.3: Multiplication of PCR products in culture and isolation of plasmids

In order to obtain cultures that multiply only a particular plasmid, per batch 8 individual colonies were taken with sterile pipette tips from the plates obtained in chapter 2.7.2, and used for inoculating in each case 1.5 ml LB/amp medium. These were cultivated overnight at 37°C on the shaker.
After completion of cultivation, 1 ml per culture was taken, transferred into a clean 1.5 ml tube or - depending on the number of samples - into a 96-well plate. The cells were then centrifuged off for 15 minutes at 4°C and 3,000 rpm, and the supernatant fluid was removed. Subsequently, plasmid isolation was carried out with the QIAprep 8 Turbo Miniprep Kit according to the instructions, using the QIAvac 6S apparatus as described below.
1. Resuspend the bacterial pellet in 250 µl P1 buffer through vortexing.
2. Add 250 µl P2 buffer per tube for lysis of the bacteria, and mix through careful inverting for 4 minutes.
3. Add 350 µl N3 buffer to precipitate the proteins and mix through careful inverting for 4 minutes.
4. Set up the QIAvac 6S apparatus.
5. Transfer the supernatant fluids into the turbo filter strip in the upper plate and apply the vacuum until all the samples have run into the columns underneath.
6. Remove the turbo filter strip and place the column strip with samples in the upper plate, replace the strip holder in the base plate by the collecting trough.
7. Apply the vacuum until all the liquid has passed through the column.
8. Add 1 ml PB buffer to wash the columns and apply the vacuum until the liquid has passed through the column.
9. Add 1 ml PE buffer to wash the columns and apply the vacuum until the liquid has passed through the column. Repeat this step once.
10. Apply the maximum vacuum for 5 minutes to dry the column membranes, then remove the entire upper plate and dry the column outlets with a highly absorbent paper towel.
11. Replace the collector trough with a 96-well plate, which has been raised through microtube stands placed underneath.
12. Fit the upper plate into the apparatus and add 100 µl EB buffer per column, incubate for 1 minute at room temperature.
13. To elute the samples, apply maximum vacuum for 5 minutes.

### Example 4.4: Restriction digestion of the isolated plasmids

In order to separate the inserts from the bacterial vector, and to check them, restriction digestion was carried out according to the following pipetting schema:
10 µl plasmid
1.5 µl 10x EcoR I buffer
0.15 µlBSA
1 µl EcoR I
2.5 µl H₂O

The restriction digestion was incubated for 1 hour at 37°C, and subsequently checked with a 1% agarose / EtBr gel.

### Example 5: Sequencing the multiplied PCR products

In order to be able to examine the PCR products which had been amplified by means of bacterial plasmids for chymase sequences, their sequences were now established.

### Example 5.1: Sequencing of selected plasmids

Due to the restriction digestion, it was possible to identify batches with different inserts, so that we did not sequence identical inserts several times over. For the sequencing, it was first of all necessary to carry out a chain termination synthesis with fluorescence-marked ddNTPs. These were available together with the reaction buffer and the normal dNTPS in the form of a ready mix called Big Dye.
The following volumes were used for the sequencing:
5 µl plasmid solution
5 µl Big Dye
1 µl primer M13-R

The primer M13-R was used for the sequencings, since this binds immediately outside the multicloning site, and thus each insert could be sequenced completely independently of its type.

The chain termination synthesis proceeded according to the following program:
- 95°C: 2 minutes

95°C 30 seconds
45°C 30 seconds 40 cycles
60°C 4 minutes

- 4°C: storage

Subsequently the batches were freed of the remaining fluorescent dyes by means of the Dye Ex 2.0 kit, according to the instructions, as these would interfere with the sequencing.
1. Vortex the Dye Ex columns with gel material for 30 seconds.
2. Open the closure by a ¼ turn of the lid.
3. Break off the closure of the column outlet by bending (not twisting).
4. Place the columns in the collecting container and centrifuge for 3 minutes at 3,000 rpm.
5. Transfer the columns into clean 1.5 ml tubes.
6. Apply the samples to the centre of the oblique gel surface without touching it with the tip of the pipette.
7. Centrifuge the columns for 3 minutes at 3,000 rpm to elute the samples.

The purified samples were then sequenced automatically using a sequencer, and the base sequences that were obtained were compared via a database search with all known DNA molecules. Furthermore, likewise via a database search, from the DNA sequences that were obtained, the corresponding amino acid sequences of the resulting proteins were determined.

### Example 6: Search for chymase coding sequences in RNA with specific primers

### Example 6.1: Production of guinea pig sequence specific primers

With the aid of the first DNA part-sequence from guinea pigs, primers specific to this were to be developed. For this, those parts of the guinea pig sequence were selected that differed most clearly from the sequences of the comparison DNA of hamsters and humans, and primers of a 30 base length were developed. These primers were then produced by a contract firm.

**Table 6: Specific primers for guinea pig chymase**

| **Primer name** | **Sequence** | **Transcription direction** |
|---|---|---|
| CHYGP_22_F | GAGAAAGACTGTAGTGGTTTCTTGATAC | Forward (seq ID 13) |
| CHYGP_23_R | GTATCAAGAAACCACTACAGTCTTTCTC | Reverse (seq ID 14) |
| CHYGP_24_F | ATGCTGCTTCTTCCTCTCCCCCTG | Forward (seq ID 15) |

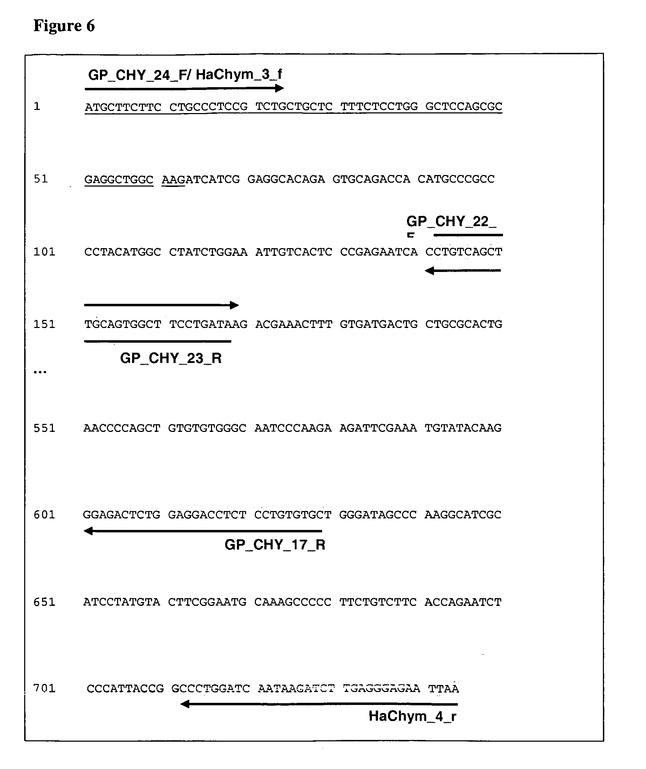

The primers used for the subsequent PCRs are shown in figure 6.

### Example 6.2: PCR with different primers

The task was once more to carry out a wide-ranging search for chymase sequences in guinea pig RNA. For this, the most varied primers that had been developed or used to date were combined with one another; amongst other things, hamster chymase primer was used again, and also the oligo (dT) primer as well as the universal adapter primer (UAP, Invitrogen). The cDNA solutions that were created with the primers oligo (dT) and adapter primer were used as templates. For the batches F and J, only the adapter primer template was used, and for the batches G and K the oligo (dT) primer template was used. All other batches were produced for both templates. In addition, batches G and K were exposed to an annealing temperature of 45°C, whereas for the others this was 58°C.

Batches were prepared with these primer combinations:

| | |
|---|---|
| A: | HaChym_3_f + GP_CHY_17_R |
| B: | GP_CHY_12_F + HaChym_4_r |
| C: | GP_CHY_15_F + HaChym_4_r |
| D: | GP_CHY_22_F + GP_CHY_17_R |
| E: | GP_CHY_22_F + HaChym_4_r |
| F: | GP_CHY_22_F + UAP Primer |
| G: | GP_CHY_22_F + Oligo (dT) Primer |
| H: | GP_CHY_24_F + GP_CHY_17_R |
| I: | GP_CHY_24_F + HaChym_4_r |
| J: | GP_CHY_24_F + UAP Primer |
| K: | GP_CHY_24_F + Oligo (dT) Primer |
| L: | GP_CHY_24_F + GP_CHY_23_R |
| M: | HaChym_3_f + GP_CHY_23_R |

The batches were composed as follows:
1 µl cDNA template
1 µl 1^{st} primer
1 µl 2^{nd} primer
20 µl HotStarTaq Master Mix
17 µl H₂O

The PCR proceeded according to the following program:
- 95°C: 10 minutes

95°C | 30 seconds
58 or 45°C | 30 seconds | 30 cycles
72°C | 3 minutes

- 72°C: 10 minutes
- 4°C: storage

The PCR batches were checked with a 1% agarose / EtBr gel.

### Example 7: Investigation and multiplication of selected PCR products

In order to have sufficient material available for investigation, selected promising PCR products were to be multiplied via transformation and bacterial cultures, and subsequently investigated for possible guinea pig sequences. For this, the batches F_{AP}, J_{AP}, H_{dI}, H_{AP}, I_{dI}, I_{AP}, L_{dI} and L_{AP} were selected.

### Example 7.1: Amplification through TOPO TA cloning and plasmid isolation

The PCR products that were obtained and were purified by means of the QIAquick Purification Kit were once again incorporated into bacterial vectors by means of the TOPO TA cloning kit, and introduced into competent cells by means of transformation. The resulting LB/amp plates were incubated overnight at 37°C in the incubator.

### Example 7.2: Colony PCR and bacterial cultures

For each batch, 6 individual colonies were taken from the plates resulting from item 2.10.1, by means of sterile pipette tips. These were first of all dipped several times into a PCR batch. In the course of this, bacteria were released which in the subsequent PCR were killed off and disintegrated in the first 95°C step, so that the plasmids contained in them could serve as templates.

The batches that were produced were composed as follows:
1 µl primer M13-F
1 µl primer M13-R
5 µl HotStarTaq Master Mix
3 µl H₂O

The PCR proceeded according to the following program:
- 95°C: 15 minutes

95°C | 30 seconds | 30 cycles
45°C | 30 seconds
72°C | 1 minute 30 seconds

- 72°C: 5 minutes
- 4°C: storage

Furthermore, the tips were then used as usual to inoculate in each case 1.5 ml LB/amp medium in a 48-well plate.
Cultivation of the liquid cultures took place overnight at 37°C on the shaker. Subsequently, the plasmids were isolated as described above, using the QIAprep 8 Turbo Miniprep Kit.

### Checking the colony PCR

Through the colony PCR, carried out in parallel with the cultivation, the intention was to make it possible at an early stage to make a statement about the inserts contained in the liquid cultures, and thus to be able to make a pre-selection of the cultures to be processed further. The results of the colony PCR were investigated by means of a 1% agarose /EtBr gel.

### Example 7.3: Sequencing of selected plasmids

Plasmids were isolated by means of the QIAprep 8 Turbo Miniprep Kit.
For the sequencing of the plasmids from the batches listed above, it was necessary to carry out chain termination synthesis with fluorescent-marked ddNTPs beforehand.

The batches are composed as follows:
5 µl plasmid solution
5 µl Big Dye
1 µl primer M13-R

The sequencing PCR proceeded according to the following program:
- 95°C: 1 minute

95°C 30 seconds
45°C 30 seconds 40 cycles
60°C 4 minutes

- 4°C: storage

Subsequently, the batches were freed of the remaining fluorescent dye by means of the Dye Ex 2.0 kit, as these would interfere with the sequencing. The purified samples were then sequenced automatically by means of a sequencer, and the sequences that were obtained were compared via a database search with all known DNA molecules.

Furthermore, likewise via a database search, from the DNA sequences that were obtained, the corresponding amino acid sequences of the resulting proteins were determined.

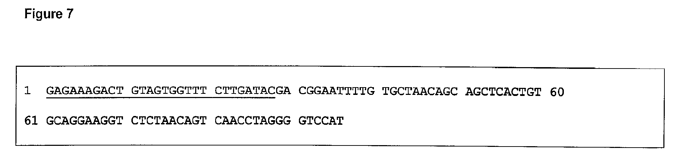

The guinea pig sequence thus obtained is shown in figure 7.

### Example 8: Completion of the sequence of guinea pig chymase

The sequences of putative guinea pig chymase that had been discovered and sequenced so far corresponded, in the case of the already fully known sequence for hamster chymase, roughly to the nucleic acid sequence that codes mature chymase. The task was now to determine, starting out from this part sequence, the rest of the chymase in the 5' direction. This was to be carried out via a so-called 5' RACE.

### Example 8.1: Developing new primers

New specific primers were selected on the basis of the newly determined sequence (table 7). These primers were produced by a contract firm.

**Table 7: Primers developed**

| **Primer name** | **Sequence** | **Transcription direction** |
|---|---|---|
| GP_CHY_25_F | CTAACAGTCAACCTAGGGGT | Forward (seq ID 16) |
| GP_CHY_26_F | AGCTCACTGTGCAGGAAGGT | Forward (seq ID 17) |
| GP_CHY_27_F | GACGGAATTTTGTGCTAACA | Forward (seq ID 18) |
| GP_CHY_28_R | ACCCCTAGGTTGACTGTTAG | Reverse (seq ID 19) |
| GP_CHY_29_R | ACCTTCCTGCACAGTGAGCT | Reverse (seq ID 20) |
| GP_CHY_30_R | TGTTAGCACAAAATTCCGTC | Reverse (seq ID 21) |

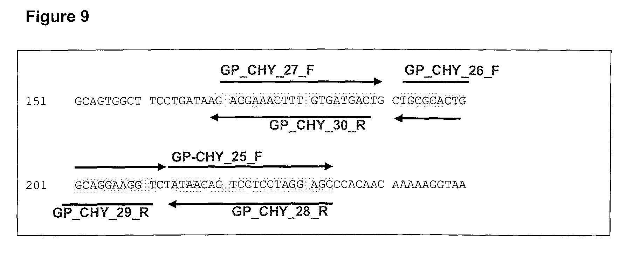

The position of the primers is shown in figure 9.

### Example 8.2: Production of different cDNA templates

In order to achieve the best possible results and to be able to exclude any degeneration of the templates, 4 different cDNA templates were produced through the use of two different kits, as described below.

### Transcriptor First Strand cDNA Synthesis Kit

1. Pipette together 2x:
   2 µl RNA 30/1 (see chapter 3.1)
   2 µl random primer (cDNA 1) or adapter primer (cDNA 2)
   9 µl H₂O
2. Incubate the mixture for 10 minutes at 65°C for adding the primers.
3. Cool on ice for 2 minutes.
4. Per batch, add:
   4 µl reaction buffer
   0.5 µl *RNase* Inhibitor
   2 µl dNTPs
   0.5 µl *reverse transcriptase*
5. Incubate the batches for 1 hour at 50°C for the procedure of the *reverse transcriptase* reaction.
6. Inactivate the *reverse transcriptase* for 5 minutes at 85°C.
7. Cool the batches on ice.

### 5' RACE Kit for Rapid Amplification of cDNA Ends

1. Producing cDNA:
- Pipette together 2x:
   0.25 µl 20 µM GP_CHY_28_R
   8 µl RNA 30/3 and 30/4 (see chapter 3.1)
   7.25 µl H₂O
- Incubate for 10 minutes at 70°C for adding the primers.
- Cool on ice for 1 minute.
- Per batch, add:
   2.5 µl 10x PCR buffer
   2.5 µl MgCl₂ solution
   1 µl dNTPs
   2.5 µl DTT
- Pre-heat for 1 minute at 42°C.
- Add 1 µl SS II reverse transcriptase per batch.
- Incubate for 50 minutes at 42°C for the procedure of the *reverse transcriptase* reaction.
- Inactivate the *reverse transcriptase* for 15 minutes at 70°C.
- Incubate for 1 minute at 37°C.
- Add 1 µl RNase mix per batch.
- Incubate for 30 minutes at 37°C to break down the RNA
- Store batches on ice.
2. S.N.A.P. purification of the batches:
- Add 120 µl binding buffer per sample
- Transfer the samples into the S.N.A.P. columns, centrifuge for 20 seconds at 13,000 rpm and 4°C
- For safety, store the through-flow on ice
- Add 400 µl cold 1x washing buffer per column, centrifuge off, and discard the through-flow. Repeat this procedure 3x.
- Wash the columns 2x with 400 µl cold 70% EtOH, and discard the through-flow.
- Centrifuge for 1 minute at 13,000 rpm and 4°C to dry the membrane.
- Transfer the columns into fresh tubes, add in each case 50 µl water that has been preheated to 65°C.
- Centrifuge for 20 seconds at 13,000 rpm to elute the cDNA (cDNA 3).
3. Appending a polycytosine tail to a part of the cDNA 3 via dC tailing:
• Pipette together:

| | |
|---|---|
| 6.5 µl | DEPC treated water |
| 5 µl | 5x tailing buffer |
| 2.5 µl | 2 mM dCTP |
| 10 µl | purified cDNA |

• Incubate for 2 minutes at 94°C
• Cool on ice for 1 minute.
• Add 1 µl TdT (name) per batch.
• For the procedure of the dC tailing, incubate for 10 minutes at 37°C.
• Inactivate the TdT for 10 minutes at 65°C.
• Cool the batches on ice (cDNA 4).

### Example 8.3: PCR to multiply the newly obtained cDNA fragments

As the quantities of cDNA that had been obtained were too small for further-reaching investigations, they were multiplied via the PCR described below.

For the PCR, the volumes listed here were pipetted together on ice:

| | |
|---|---|
| 34.4 µl | H₂O |
| 5 µl | Expand High Fidelity PCR buffer + MgCl₂ |
| 1 µl | dNTPs |
| 2 µl | 1^{st} primer |
| 2 µl | 2^{nd} primer |
| 5 µl | cDNA |
| 0.6 µl | Taq polymerase (3.5 U/µl) |

These primer combinations were used here:

| | |
|---|---|
| cDNA 1: | GP_CHY_17_R + 27_F |
| cDNA 2: | GP_CHY_17_R + 27_F and UAP + GP_CHY_27_F |
| cDNA 3: | GP_CHY_24_F + 29_R |
| cDNA 4: | GP_CHY_24_F + 29_R and UAP + GP_CHY_29_R |

For the batch with the cDNA 2, in which the UAP was used, the 3' RACE took place, and for the batch with cDNA 4 correspondingly the 5' RACE.

The PCR proceeded according to the following program:
- 95°C: 10 minutes

95°C 30 seconds
55 30 seconds 35 cycles
72°C 3 minutes

- 72°C: 10 minutes
- 4°C: storage

The samples were then checked with a 1% agarose / EtBr gel.

### Example 8.4: Nested amplification

Since simple PCR was mostly insufficient for producing visible amounts in this obtaining of cDNA, a further PCR was carried out, which in this connection was referred to as "nested amplification".

For this, the following volumes were pipetted together:

| | |
|---|---|
| 36.4 µl | H₂O |
| 5 µl | Expand High Fidelity PCR buffer + MgCl₂ |
| 1 µl | dNTPs |
| 1 µl | 1^{st} primer |
| 1 µl | 2^{nd} primer |
| 5 µl | cDNA from PCR 1 (diluted 1:100) |
| 0.6 µl | Taq polymerase (3.5 U/µl) |

These primer combinations were used here:

| | |
|---|---|
| cDNA 1: | GP_CHY_17_R + 26_F (1) and GP_CHY_17_R + 25_F (2) |
| cDNA 2-17: | GP_CHY_17_R + 26_F (3) and GP_CHY_17_R + 25_F (4) |
| cDNA 2-UAP: | UAP + GP_CHY_26_F (5) and UAP + GP_CHY_25_F (6) |
| cDNA 3: | GP_CHY_24_F + 30_R (7) |
| cDNA 4-24: | GP_CHY_24_F + 30_R (8) |
| cDNA 4-UAP: | GP_CHY_24_F + 30_R (9) and UAP + 30_R (10) |

The amplification products were subsequently checked with a 1% agarose / EtBr gel.

The products of the nested amplification are subsequently cloned, as described above, multiplied and sequenced.

### Example 9: Expression of recombinant guinea pig chymase

Recombinant guinea pig chymase is expressed by means of the cloned sequence (Seq ID No. 1) in a suitable expression system, e.g. Pichia pastoris, and purified (Nakabuko et al., 2000, Yeast 16(4), 315-23; Takai et al., 1997, Clin. Chim. Acta265, 13-20; Schechter et al., 1993, J. Biol. Chem.268, 23626-23633).

### Example 9.1: Expression & Purification of Guinea Pig Chymase

The recombinant guinea pig chymase was expressed in BL21(DE3) cells as an N-terminal fusion protein with a 26-amino acid polypeptide carrying a 6xHis tag and a enterokinase cleavage site. Since the protein was expressed forming insoluble inclusion bodies it had to be renatured. Isolated and purified inclusion bodies were modified under denatured conditions with glutathione and then dissolved in 6 M guanidine·HCl, 20 mM EDTA pH 4.5. Refolding was done by a 1:100 dilution of the protein solution into a large volume of refolding buffer: 50 mM Tris/HCl pH 8.0, 750 mM arginine, 1 mM EDTA, 2 mM cysteine. Alkaline pH was chosen to promote thiolate anion formation and hence disulphide exchange. The preparation was kept at 4°C for two days. Caused by the high ionic strenght of the refolding solution the whole preparation had to be concentrated by ultrafiltartion and dialysed prior to a first chromatography. The dialysis buffer contained 50 mM Tris/HCl, 300 mM NaCl and 1 mM □m̃ercaptoethanol. Raw and still inactive chymase could be obtained by a chromatography on Ni-NTA using the same buffer and a gradient of imidazole to elute the protein from the column. Then the enzyme was activated by cleaving off the N-terminal 6xHis tag with enterokinase. Thereby the mature guinea pig chymase with the correct N-terminus was formed. However, beside activation partial self digestion of the protein was also observed. The activated enzyme was twice chromatographed on Superdex 75, first in 50 mM Tris/HCl pH 8.0, 300 mM NaCl, 1 mM TCEP, 10% glycerol and then in 50 mM MES, 300 mM NaCl, 1 mM TCEP, 10% glycerol in a slightly acidic pH of 5.5 because chymase is described to be inactive at that pH (corresponds to the pH in mast cells, where active chymase is stored). Finally, as shown by SDS-PAGE >98% pure protein was obtained. The purified protein was monomeric as detected by analytical ultracentrifugation and highly active in a fluorometric assay.

### Example 10: Chymase activity assays

The activity of guinea pig chymase is measured using familiar methods (Muramatsu et al., 2000, J. Biol. Chem. 275, page 5546; Uehara et al., 1999, Hypertension 35, page 57; Takai et al., 1997, Clin. Chim. Acta 265, p. 15).

### Example 10.1: Assay for Chymase inhibition

For the chymase a substrate was chosen containing the 4 amino acid peptide AAPF as a standard substrate for chymotrypsin like compounds (succinyl-Ala-Ala-Pro-Phe-[7-amino-4-methylcoumarin]; Lockhart BE, et al., "Recombinant human mast-cell chymase: an improved procedure for expression in Pichia pastoris and purification of the highly active enzyme." Biotechnol Appl Biochem. published as immediate publication 26 May 2004 as manuscript BA20040074)). The peptide was synthesized with a purity of 95% from Bachem, Bubendorf, Switzerland). Chymase purified form human skin mast cells was obtained from Calbiochem (Merck Biosciences, San Diego, California, USA). The assay buffer was 0.15 M NaCl, 0.05M, Tris HCl, 0.05% CHAPS, 0.1mg/ml Heparin (Heparin sodium, Sigma, porcine intestinal mucosa), 0.02mM AAPF-substrate, 1nM Chymase at pH 7.4. The assay was performed in 96-well plates (Packard Optiplate), with a 0.05ml volume at room temperature. Chymase activity was indicated by the initial rate of increase in fluorescence at 340/440nm (excitation / emission) from free 7-amino-4-methylcoumarin released from the substrate. Inhibition of the activity by inhibitory compounds was read after 30 min pre-incubation with the chymase at room temperature in assay buffer without AAPF-substrate. The assay was then started by addition of the indicated concentration of AAPF-substrate.

**Table 8**

| Inhibition of guinea pig chymase activity: | | | |
|---|---|---|---|
| IC50 nM GP chymase | human chymase coli | | |
| **940** | **570** | ref cpd | Meiji Seika Kaisha SF2809-V |
| 345 | 590 | ref cpd | Toa Eiyo TY-51076 |

## Claims

1. Nucleic acid sequence comprising the sequence of Seq ID No. 1.

2. Nucleic acid sequence according to claim 1, wherein the nucleic acid sequence is the sequence according to Seq ID No. 1.

3. Polypeptide sequence comprising the sequence of Seq ID No. 2.

4. Polypeptide sequence according to claim 3, wherein the polypeptide sequence is the sequence according to Seq ID No. 2.

5. Nucleic acid sequence comprising a nucleic acid sequence that encodes the polypeptide sequence according to claim 3 or 4.

6. Expression vector, comprising the nucleic acid sequences according to claim 1, 2 or 5.

7. Host cell, comprising the expression vector according to claim 6.

8. A guinea pig with somatic cells and germ cells with a mutated chymase gene, wherein at least one allele is functionally interrupted through homologous recombination of the mutated chymase gene, wherein this functional interruption inhibits the expression of the functional chymase gene in the guinea pig cells.

9. Method for screening compounds that modulate the activity of chymase, comprising the steps: a) bringing a compound into contact with isolated polypeptide according to claim 3 or 4; and b) determining the activity of guinea pig chymase; wherein a compound that inhibits or stimulates the activity of the guinea pig chymase is a compound that modulates the activity of the guinea pig chymase.

10. Method for identifying compounds that modulate the activity of chymase, comprising the steps: a) bringing a compound into contact with the host cell according to claim 7; and b) determining the activity of the guinea pig chymase that is expressed in the host cell; wherein a compound that inhibits or stimulates the activity of the guinea pig chymase is a compound that modulates the activity of the guinea pig chymase.

11. Method for identifying compounds that modulate the activity of chymase, comprising the steps: a) administering a compound to guinea pigs; and b) determining the activity of the guinea pig chymase; wherein a compound that inhibits or stimulates the activity of the guinea pig chymase is a compound that modulates the activity of the guinea pig chymase.

12. Method for identifying compounds that inhibit PAOD, comprising the steps: a) administering a compound to guinea pigs; b) causing PAOD, and c) determining the extent of PAOD; wherein a compound that inhibits PAOD is a compound that leads to a reduction in the extent of the PAOD that is caused by step b).

13. The method according to claim 12, wherein PAOD is caused by administering lauric acid, through damage to a vessel with a balloon catheter or by feeding a diet with a high proportion of fat.

14. Method for identifying compounds to combat asthma, comprising the steps: a) administering to guinea pigs a compound that inhibits the guinea pig chymase; b) causing asthma; and c) determining the extent of asthma, wherein a compound that inhibits asthma is a compound that leads to a reduction in the extent of the asthma that is caused by step b).

15. The sequences, methods and guinea pigs as described above, in particular in relation to the preceding examples.
